# EUROPEAN PATENT SPECIFICATION

(11) **EP 1 439 796 B1**
(45) Date of publication and mention of the grant of the patent: **07.04.2010**
(21) Application number: 02785288.8
(22) Date of filing: 23.10.2002
(51) Int. Cl.: A61F 2/00

(54) **AREAL IMPLANT**
FLÄCHIGES IMPLANTAT
IMPLANT AREOLAIRE

(30) Priority: 29.10.2001 DE 10153334
(43) Date of publication of application: 28.07.2004
(73) Proprietor: Johnson & Johnson Medical GmbH, 22851 Norderstedt (DE)
(72) Inventor: UGAHARY, Franz, NL-4002 WP Tiel (NL); SCHULDT-Hempe, Barbara, 24576 Bad Bramstedt (DE); PRIEWE, Jörg, D-24114 Kiel (US); WALTHER, Christoph, 24568 Kattendorf (DE)
(74) Representative: UEXKÜLL & STOLBERG
(86) International application number: PCT/EP2002/011860
(87) International publication number: WO 2003/037215

(56) References cited:
- WO-A-01/54589
- WO-A-01/56499
- WO-A-02/07648
- DE-A- 19 832 634
- US-A- 5 122 155

## Description

The invention relates to an areal implant having a mesh-like basic structure and a marking in the central area.

The repair of inguinal hernias with the help of an implant mesh is widespread. In 1995, an operation procedure was developed by F. Ugahary which combines the advantage of a preperitoneal mesh fixation with the convenience of the so-called small gridiron incision as access (Volker Schumpelick, "Hernien", 4th edition, Thieme-Verlag, pp 221). A non-resorbable plastic mesh is in particular suitable as an implant. It is marked in the middle with a cross made of resorbable suture material. This central marking facilitates the positioning of the implant during the operation. The Ugahary procedure is carried out in open surgery.

In addition, there are also laparoscopic techniques for repairing inguinal hernias. One example is transabdominal preperitoneal mesh-plasty (TAPP), where an implant mesh is preperitoneally positioned through a transabdominal laparoscopic access (loc. cit., pp 224). Another technique is totally extraperitoneal preperitoneal mesh-plasty (TEP), in which a large mesh is preperitoneally laparoscopically applied via an extraperitoneal access and covers all three potential hernial openings (loc. cit., pp 229).

Cicatricial hernias can also be treated by means of implant meshes, e.g. with the help of preperitoneal mesh-plasty (PNP) (loc. cit., pp 281).

Suitable implant meshes are described e.g. in DE 196 13 730 A1 (corresponding to EP 0 797 962 A1) or DE 199 42 611 A1 (corresponding to WO 01/15625).

Although a marking in the central area, as in the case of the Ugahary technique, is helpful in the positioning of an implant mesh, the performance of the surgery nevertheless requires some skill.

The object of the invention is to provide a possibility of facilitating the course of the surgery when inserting an implant mesh, in particular when repairing hernias.

This object is achieved by an areal implant with the features of claim 1. Advantageous designs of the invention emerge from the dependent claims.

The areal implant according to the invention has a mesh-like basic structure and a marking in the central area (which can also be arranged outside the geometric centre). A marking line runs through this central marking.

The marking line is preferably straight, and it extends to the edges of the implant. The implant can also have further marking lines. In a preferred version, on each side of the marking line running through the central marking a further marking line is provided which preferably runs parallel to the marking line running through the central marking; the two further marking lines are preferably equidistant from the marking line running through the central marking. The marking line running through the central marking can have a greater width than the further marking lines.

In the case of a typical application of the implant according to the invention, the mesh-like basic structure has a rectangular shape and is provided in the centre with a marking through which the marking line runs parallel to the longer sides of the rectangle. A further marking line is situated on each side of this marking line. Primarily non-resorbable materials or mixtures of non-resorbable and resorbable materials are suitable for the basic structure, and preferably resorbable material for the markings (see below also). In the case of this application, the implant serves to support the tissue and permanently stabilize the fascial structures during the open inguinal hernia repair according to the gridiron incision technique. The implant is inserted preperitoneally. After sufficient preparation, the implant should be positioned stress-, fold-, and void-free and fixed with at least one suture using non-resorbable suture material (e.g. of polypropylene). To make possible a correct positioning of the implant according to the gridiron incision technique, the skin in the operating area should be adequately marked. The skin markings and the markings provided on the implant facilitate the identification of the relevant structures and the implantation. The central marking indicates the middle of the implant and must lie medially relative to the epigastric vessels in the example, in order that the mesh-like basic structure adequately overlaps the fascial defect on all sides, in order to also adequately stabilize the fascial edges. The marking line running through the central marking (caudal auxiliary line) is aligned parallel to the inguinal belt. In order to guarantee an optimum position, the implant as a whole should not be individually configured, but if required, the edges can be rounded off. In the case of fixing (preferably with the help of non-resorbable suture material) according to the gridiron incision technique, at least 1 cm mesh edge should be taken. If necessary and feasible, a gap of 1 cm to 2 cm should be maintained between individual sutures. At least one mesh link should be gripped for each suture.

The marking line running through the central marking therefore much facilitates the alignment of the implant, as described here using the example of the Ugahary technique. If it extends parallel to the inguinal belt and the central marking lies medially relative to the epigastric vessels, the surgeon can be sure that the implant is correctly positioned. With the help of the further markings lines, the current position of the implant can be more easily ascertained during the positioning process, so that adjustments can be carried out where appropriate. If the marking line running through the central marking is wider than the further marking lines, the individual marking lines can be better distinguished from one another.

There are a variety of possibilities for the design of the central marking and of the marking lines.

For example, the central marking and/or at least one marking line can have thread- or yarn-like material which is connected to the basic structure preferably via textile techniques or incorporated into the basic structure, e.g. by warp-knitting, weft-knitting, embroidery, sewing or knotting. Multi-step processes can be used in which the mesh-like basic structure is prepared first and the markings are then applied. However, one-step processes are particularly advantageous in which the markings are created together with the basic structure. This is explained in more detail below using examples.

In the case of a further possibility, the central marking and/or at least one marking line contains areal material which is connected to the basic structure preferably by gluing, welding or sewing. Conceivable as areal material are e.g. textile materials such as woven or knitted fabrics, but also films from which the markings are, e.g., punched out or cut out.

In the case of a further design, the central marking and/or at least one marking line contains at least one shaped body, preferably as beads or tubelets. Such beads, tubelets or similar shaped bodies, the colour of which preferably differs from that of the basic structure, can, e.g., be sewn to the basic structure with suture material.

The central marking and/or at least one marking line can also contain a colouring agent which preferably contains a dye and a binder (e.g. a polymer). In this case, printing or spraying techniques can be used in order to apply the markings to the basic structure. The colouring agent can be prepared, e.g., by dissolving dye and polymer in a suitable solvent and sprayed onto the basic structure with the help of an airbrush technique or an ink-jet printer. After the evaporation of the solvent, the desired markings are connected firmly to the basic structure. Other examples are stamping and painting of the markings onto the basic structure, and the evaporation of a colouring agent, the areas of the basic structure which are not intended to receive colour being covered by a screen. If a roller printing method is used, the implant can be continuously processed.

A further possibility is to use a warm mixture of a dye and a binder as colouring agent, paint the desired markings onto the basic structure and then cool to room temperature so that the binder solidifies.

In the case of a preferred version of the invention, the central marking and/or at least one marking line is feelable. Preferably, the relevant marking rises by more than 0.75 mm above the basic structure; in this case, the central marking or the marking lines can still be felt with a surgical glove. Such feelable markings can be produced, e.g., by doubling the thickness of the implant in the marking area or with the help of the shaped bodies already mentioned. Feelable markings can be of great advantage when performing a surgery, e.g. when using the Ugahary technique.

The marking lines can be constructed as a continuous line or an interrupted line, the width preferably being in the range from 0.3 mm to 8.0 mm.

Many different shapes are conceivable for the central marking, e.g. a circle, a rhombus or a polygon, but also shapes indicating a direction (e.g. an arrow). Shapes such as, e.g., a rhombus can be created, e.g., with the help of knitting techniques, as explained in more detail below using an embodiment.

Whilst it is possible in principle for the markings to have the same colour as the basic structure (e.g. in the case of feelable markings), the central marking and/or at least one marking line preferably contains a dye, so that the colour of the central marking and/or the at least one marking line differs from that of the basic structure. A dye was already mentioned above in connection with a colouring agent, but the explained thread- or yarn-like materials or the areal materials for the central marking or the marking lines are also preferably coloured. Examples of dyes are organic dyes, fluorescent dyes, inorganic dyestuff pigments, titanium dioxide, zirconium dioxide, iron pigments, magnetite, or mixtures of such dyes; see also below.

A variety of designs are conceivable for the basic structure, e.g. knitted fabrics or knitted fabrics with additional reinforcing structures. Examples thereof can be found in the publications mentioned at the outset and in the literature relating to the state of the art.

Numerous non-resorbable, slowly resorbable or resorbable polymers or copolymers can be considered as materials both for the basic structure and for the central marking and the marking lines, as listed in detail in the claims.

Particularly suitable materials for the mesh-like basic structure are monofilaments or multifilaments of polypropylene or Pronova^{®} (Ethicon). Pronova^{®} is a mixture of polyvinylidene fluoride and copolymers of vinylidene fluoride and hexafluoropropene. Mixtures or composites, also e.g. in the form of multifilaments, of these materials are likewise very suitable.

Examples of preferred resorbable materials are poly-p-dioxanone and Vicryl^{®} (Ethicon). Vicryl^{®} is a copolymer of L-lactide and glycolide in the ratio 10:90. Such resorbable components can also be contained, e.g. in the form of filaments, in multifilament composite materials which additionally contain filaments of non-resorbable materials.

In the case of preferred versions, the central marking and the marking lines contain resorbable material. Examples of a yarn-like material for the markings are a fourfold twist of 80 den Vicryl^{®} (violet), an eightfold twist of 80 den Vicryl^{®} (violet) or a composite of 1x polypropylene (blue) and 8x Vicryl^{®} (violet). Resorbable materials for the markings have the advantage that they have decomposed some time after the implantation and can no longer bother the patient. Non-resorbable portions are, on the other hand, still visible after a possibly necessary explantation and thus allow better monitoring.

The selection of a suitable dye depends on the base material concerned. In the following Tables 1 and 2, examples of dyes with which the respective base materials can be coloured are given, respectively, for resorbable and non-resorbable base materials.

In a particularly preferred version, the central marking and the marking lines contain a copolymer of L-lactide and glycolide in the ratio 10:90 (i.e. Vicryl^{®}), which is coloured with 1-hydroxy-4-p-toluidino-anthraquinone. Multifilament yarn with these components is marketed by Ethicon under the name "Vicryl, violett". Vicryl^{®} is a material which is wetted very swiftly by tissue fluids. If a multifilament yarn of the "Vicryl, violett" type is used in a surgery, this yarn is wetted and penetrated almost immediately by tissue liquid or blood so that the markings show up very clearly and almost black. In this way, a very strong coloration is obtained with relatively little use of dye.

Non-resorbable materials such as polypropylene or Pronova^{®} are wetted much more slowly and not penetrated by body fluids, so that the visibility of non-resorbable markings which contain such materials and an admixed or embedded dye depends greatly on the dye proportion. Preferably, the quantity of the dye which remains permanently or at least a long time in the patient's body is chosen to be as small as possible. Therefore, the preferably used dye proportions correspond to the dye proportions or concentrations customarily used in non-resorbable suture material.

The dyes or dyestuff pigments used (see above) are usually mixed with the polymer of the base substance or a binder and optionally bound therein or embedded in a polymer matrix. Among the dyestuff pigments, e.g. white pigments such as titanium dioxide and zirconium dioxide or yellow iron pigments are to be emphasized. Pigments which contain an element with a high atomic number are X-ray-opaque; the use of such dyestuff pigments therefore makes it possible to locate the implant or its markings after the surgery with the help of X-ray diagnostic procedures. The use of magnetite (brown) leads to a magnetic-resonance-active material.

The areal implant provided with the central marking and the at least one marking line is preferably packed and sterilized by the manufacturer. It can therefore be used immediately in a surgery and can be implanted under sterile conditions. Thus, there is no risk that the implant may be contaminated upon application of a marking (which, with the conventional Ugahary technique, is done in the operating theatre).

**Table 1 Resorbable materials with dyes**

| **Material** | **Trade name** | **Dye** |
|---|---|---|
| Copolymer of L-lactide/glycolide 10/90 | Vicryl | D&C Violett No. 2 [1-hydroxy-4-p-toluidino-anthraquinone] C.I. 60725 |
| Polyglycolide (polyglycolic acid) | Dexon | D&C Green |
| Poly-p-dioxanone | PDS, blue | D&C Blue No. 6 [Indigotin] C.I. 73000 |
| Poly-p-dioxanone | PDS, violet | D&C Violett No. 2 [1-hydroxy-4-p-toluidino-anthraquinone] C.I. 60725 |
| Copolymer of L-lactide/glycolide 95/5 | Panacryl | D&C Violett No. 2 [1-hydroxy-4-p-toluidino-anthraquinone] C.I. 60725 |
| Copolymer of L-lactide/glycolide 10/90 | Vicryl Rapid | D&C Violett No. 2 [1-hydroxy-4-p-toluidino-anthraquinone] C.I. 60725 |
| Copolymer of glycolide (60%), p-dioxanone (14%) and trimethylene car-bonate (26%) | Biosyn | D&C Violett |
| Copolymer of glycolide (75%) and epsilon-caprolacton (25%) | Monocryl | D&C Violett No. 2 [1-hydroxy-4-p-toluidino-anthraquinone] C.I. 60725 |

**Table 2 Non-resorbable materials with dyes**

| **Material** | **Trade name** | **Dye** |
|---|---|---|
| Polypropylene | Prolene | Copper phthalocyanine blue C.I. 74160 |
| Mixture of polyvinylidene fluoride and a copolymer of vinylidene fluoride and hexafluoropropene | Pronova | Copper phthalocyanine blue C.I. 74160 |
| Polyester (PET) | Mersilene/ Ethibond | D&C Green No. 6 C.I. 61565 |
| Polyamide 6,6 | Suturamid, black | Indanthrene Direktschwarz R Colloisol, composed ½ each of VAT Blue 20 (C.I. 59800) and olive dye (C.I. 69515)/Palamid Black 00-6005 |
| Polyamide 6/ Polyamide 6,6 | Ethilon, black | Hematoxylin |
| Polyamide 6 | Ethilon, blue | Pigment Blue 9860/ Chromophtal Blue A3R C.I. Pigment Blue 60 |
| Polyamide 6,6 | Nurolon, black | Logwood extract (hematin), black C.I. 75290 |

The manner in which the areal implant according to the invention can advantageously be used in a surgery according to the Ugahary technique has already been explained. Here is a summary: When repairing an inguinal hernia in the gridiron incision technique according to Ugahary, the markings make the correct positioning of the implant easier. The central marking should lie medially relative to the epigastric vessels in order to guarantee an adequate overlapping of the defect on all sides. Furthermore, with a correct positioning, the marking line running through the central marking (caudal auxiliary line) of the implant runs parallel to the inguinal belt. Any additional marking lines which are provided parallel to the marking line running through the central marking are aligned parallel to the inguinal belt. The hernia and other possible hernial openings are then optimally covered. The surgery takes place in the restricted working space with feelable and optical monitoring.

The implant according to the invention can also be used in laparoscopic procedures such as TEP or TAPP (see above). The central marking is designed such that it can be easily recognized endoscopically. During the surgery, the position of the implant is constantly visually monitored, which is made easier by the central marking and the at least one marking line.

A further possible use for the implant according to the invention is preperitoneal mesh-plasty (PNP), a process for the repair of cicatricial hernias (Volker Schupelick, "Hernien", 4th edition, Thieme-Verlag, pp 281). For this, an implant with several marking lines is used, e.g. a continuous wide marking line which runs through the central marking and parallel lines running on both sides at a distance of e.g. 2 cm (one or more on each side). The marking lines make it possible for the surgeon to check the size of the implant bearing after the positioning of the implant, as a sufficiently large covering of the wound edges is necessary.

In advantageous versions, the areal implant according to the invention has at least one marking on its back face, i.e. on or related to the face of the basic structure opposite to the face considered so far. The at least one marking of the back face can comprise at least one marking line, e.g. a straight line and/or a zigzag line. Preferably, the pattern of markings on the back face is different from the pattern of markings considered before. This can facilitate the handling of the implant during a surgical procedure.

In the following, the invention is explained using further embodiments. The drawings show in
- Figure 1: a top view of the version of the implant according to the invention prepared according to Example 1,
- Figure 2: the course of the thread for the central marking of the implant from Figure 1,
- Figure 3: a top view of the version of the implant according to the invention according to Example 2, and
- Figure 4: the course of the thread for the central marking of the version of the implant according to the invention prepared according to Example 3.

### Example 1

During the preparation of the mesh-like basic structure of an areal implant as a warp-knitted product on a crochet galloon machine, additional threads were incorporated as optical marking lines. An implant marketed by Ethicon under the name "Vypro^{®} II" served as basic structure.

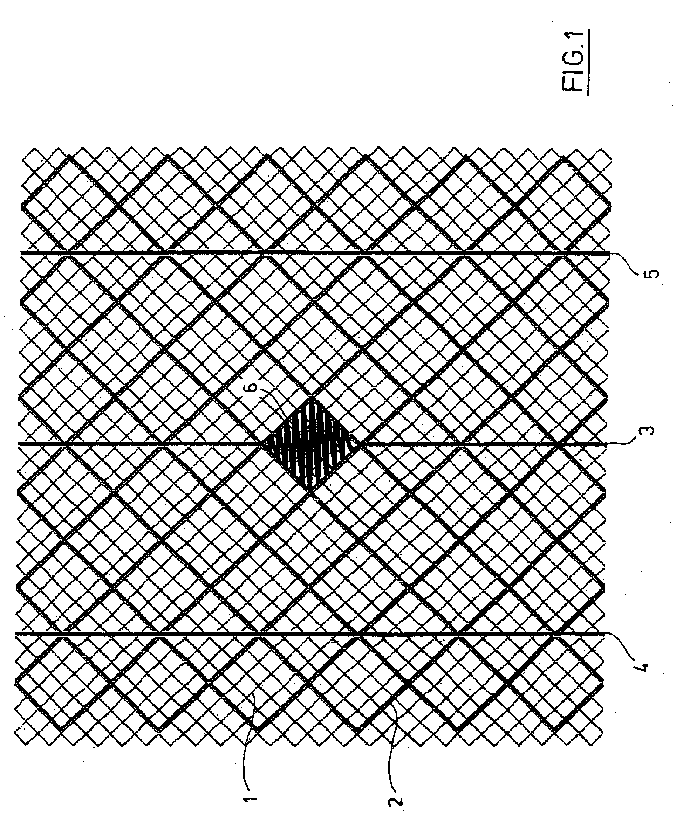

A "Vypro^{®} II" mesh consists in its base structure of approximately equal parts of resorbable multifilament yarn of Vicryl^{®} (see above) and non-resorbable multifilament yarn of polypropylene. An additional reinforcing thread (violet Vicryl^{®} with monofilament polypropylene) runs rhombically over the mesh, so that the reinforcing structure formed by the reinforcing thread has several times the pore size of the previously mentioned base structure. In Figure 1, the base structure is numbered 1 and the reinforcing structure 2. The base structure 1 and the reinforcing structure 2 together form the mesh-like basic structure of the implant.

In the embodiment, to produce marking lines 3, 4 and 5, three threads running in separately were processed with the basic structure by means of additional thread guides.

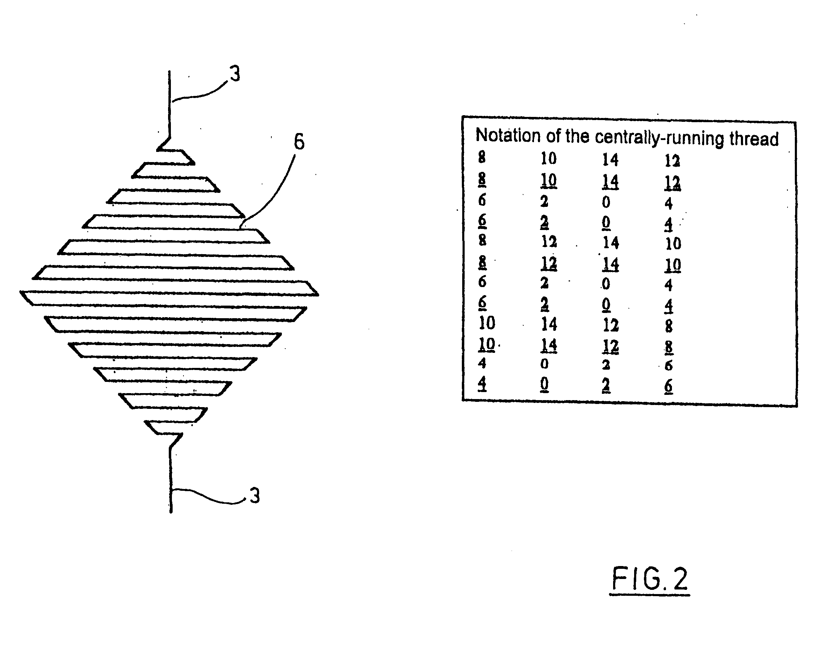

The middle thread 3, an eightfold twist of Vicryl^{®} (violet; see Table 1) forms a stationary weft and is incorporated as partial wefts at defined intervals in the form of a rhombus 6, corresponding to the rhombus size of the mesh-like basic structure determined by the reinforcing structure 2. The notation of the rhombus 6 emerges from Figure 2 in a manner understandable to a person skilled in the art. The rhombus 6 forms a central marking of the implant.

The two outer threads 4 and 5 (likewise eightfold twists of Vicryl^{®}, violet) run straight as a stationary weft in the basic structure. The distance from the thread 3 running in the middle is 16 wales.

### Example 2

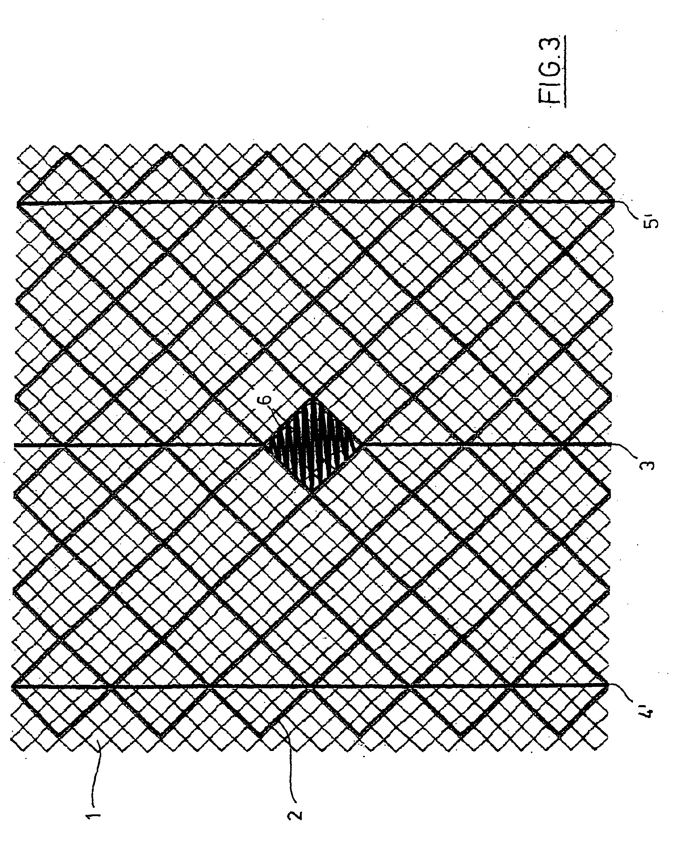

The version represented in Figure 3 of an areal implant was prepared in a similar way to the version according to Example 1, i.e. again with a "Vypro^{®} II" implant mesh customary in the trade with marking threads additionally incorporated during production.

The only difference from the version according to Example 1 is the greater distance between the two outer marking lines (which here are numbered 4' and 5') and the marking line 3 running through the central marking 6. This distance is 20 wales in each case here.

### Example 3

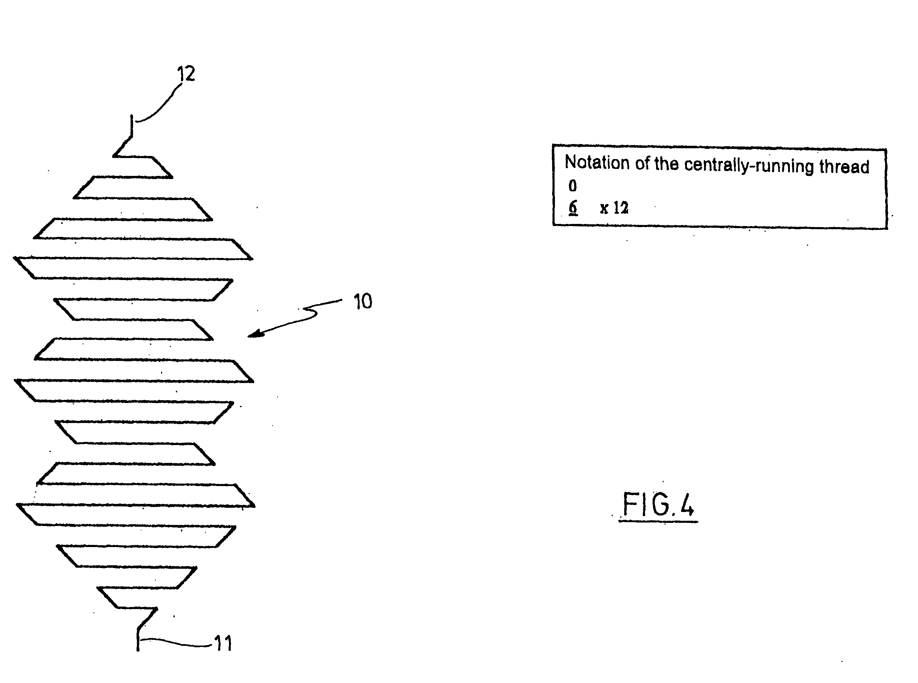

In a similar way to Examples 1 and 2, an additional thread was incorporated as marking line during the production of a "Vypro^{®} II" basic structure. This thread, an eightfold twist of Vicryl^{®}, was processed, using an additional thread guide, with the basic structure produced in the form of a "Vypro^{®} II" mesh. It forms a stationary weft and was incorporated at defined intervals as partial wefts roughly in the form of a rectangle, corresponding to the rhombus size of the "Vypro^{®} II" mesh. Details are shown in Figure 4.

The rectangle 10, which strictly speaking has a shape deviating from a rectangle, but essentially acts as a rectangle, is the central marking of the implant. The marking line running through this central marking in the middle of the implant is formed by the thread sections above and below the rectangle 10. Parts 11 and 12 thereof are shown in Figure 4. No further marking lines are provided in this version.

### Example 4

An additional thread, namely a fourfold twist of Vicryl (violet; Ethicon), was incorporated as middle-running marking line onto a mesh-like implant ("Vypro^{®} II", Ethicon) customary in the trade. For this, the thread was stitched, using an embroidery machine in the flat-stitch process, onto the weft-knitted product of the basic structure formed by the "Vypro II" mesh, the embroidery in the central area of the implant being formed as a central marking having the size of a rhombus of the reinforcing structure of the "Vypro^{®} II" mesh.

### Example 5

Additional threads, namely fourfold twists of Vicryl^{®} (violet; Ethicon), were applied in knotting technique to a surgical implant mesh of polypropylene (Prolene^{®} mesh, Ethicon) to form a central marking and a marking line running through the central marking. The threads were linked in the middle of the implant in the form of a circle.

### Example 6

A surgical mesh of polypropylene (Prolene mesh, Ethicon) customary in the trade was cut quadratically to a size of 15 cm x 15 cm as basic structure.

Blue-dyed poly-p-dioxanone, as is also used in the product Durapatch^{®} marketed by Ethicon GmbH, was extruded in a film extruder with a thickness of 120 µm. A strip measuring 0.5 cm x 10 cm and a rectangle measuring 1 cm x 2 cm were cut out therefrom and laid on the basic structure such that the strip ran through the middle of the basic structure and the rectangle was placed in the middle of the basic structure. These film pieces were pressed with a hot press accompanied by warming with the basic structure.

The implant was then packed, sterilized with ethylene oxide and the pack sealed.

### Example 7

A surgical polyester mesh (Mersilene^{®}, Ethicon) was cut to a size of 10 cm x 20 cm as mesh-like basic structure. In the example serving as a test, two film strips measuring 0.5 cm x 5 cm were attached analogously to example 6 parallel to the longitudinal direction of the basic structure at a distance of 3 cm from the edge.

Approximately 0.5 mm-thick sheets were pressed, using a hot press, from granules of poly-p-dioxanone, as are also used in the production of the film for the product Durapatch^{®} (Ethicon), and these were cut out or punched out in the form of a rhombus with a length of 2 cm and a width of 0.8 cm. This rhomboidal marking was welded from the side of the basic structure with an ultrasound probe in the middle of the basic structure, so that, in addition to the strips, an orientation-dependent central marking was obtained which displayed a thickness and rigidity different from the basic structure and was also clearly feelable with the help of surgical gloves.

### Example 8

As basic structure, a surgical implant mesh customary in the trade of polyester (Mersilene^{®}, Ethicon) was cut to a size of 15 cm x 20 cm.

13.6 mg of the dye D & C #2 violett, a dye which is also used in the commercially available resorbable material Vicryl^{®}, and 3.44 g polycaprolactondiole with a molecular weight of 2000 (manufacturer: Polysciences Inc.) were put into a small beaker and stirred at 100°C hot-plate temperature on a magnetic stirrer. The melt was dyed a homogeneous and intense blue/violet. The melt was then applied with a thin polyethylene rod to the basic structure. The marking in the central region of the basic structure was oval, had a length of approx. 1 cm and a width of 2 mm to 3 mm. The mechanical thickness measurement showed a marking thickness of 750 µm and a thickness of the mesh-like basic structure of approx. 220 µm. This marking was also easily felt with surgical gloves because of its height and different rigidity. An approximately 1 mm-wide and 10 cm-long line was drawn parallel to the mesh edge with the same melt, which was likewise intensely dyed and also easily recognizable under poor light conditions.

### Example 9

As basic structure, a resorbable surgical implant mesh customary in the trade made of Vicryl^{®} ("Vicryl-Netz Typ 9", Ethicon) was cut to a size of 15 cm x 20 cm.

12.5 mg of the dye D & C #2 violett, a dye which is also used in the commercially available resorbable material Vicryl^{®}, and 5.2 g polycaprolactondiole with a molecular weight of 2000 (manufacturer: Polysciences Inc.) were put into a small beaker and stirred at 100°C hot-plate temperature on a magnetic stirrer. The melt was dyed a homogeneous and intense blue/violet. The melt was then applied with a thin polyethylene rod to the basic structure. The marking in the central region of the basic structure was oval, had a length of approx. 1 cm and a width of 2 mm to 3 mm. The mechanical thickness measurement showed a marking thickness of 750 µm and a thickness of the mesh-like basic structure of approx. 220 µm. This marking was also easily felt with surgical gloves because of its height and different rigidity. An approximately 1 mm-wide and 10 cm-long line was drawn parallel to the mesh edge with the same melt, which was likewise intensely dyed and also easily recognizable under poor light conditions.

## Claims

1. Areal implant having a mesh-like basic structure (1, 2) and a marking (6) in its central area, a marking line (3) running through this central marking (6), wherein the marking line (3) is straight, **characterized in that** the marking line (3) extends up to the edges of the implant.

2. Implant according to claim 1, **characterized by** further marking lines (4, 5).

3. Implant according to claim 2, **characterized in that**, on each side of the marking line (3) running through the central marking (6), a further marking line (4, 5) is provided which preferably runs parallel to the marking line (3) running through the central marking (6).

4. Implant according to claim 2 or 3, **characterized in that** the marking line running through the central marking has a greater width than the further marking lines.

5. Implant according to one of claims 1 to 4, **characterized in that** the central marking (6) and/or at least one marking line (3, 4, 5) comprise thread- or yarn-like material which is connected to the basic structure (1, 2) preferably in one of the ways selected from the following group: warp-knitting, weft-knitting, embroidery, sewing, knotting.

6. Implant according to one of claims 1 to 5, **characterized in that** the central marking and/or at least one marking line contains areal material which is connected to the basic structure preferably in one of the ways selected from the following group: gluing, welding, sewing.

7. Implant according to one of claims 1 to 6, **characterized in that** the central marking and/or at least one marking line comprises at least one shaped body, preferably as a bead or tubelet.

8. Implant according to one of claims 1 to 7, **characterized in that** the central marking and/or at least one marking line contains a colouring agent, which preferably includes a dye and a binder and which is preferably applied in one of the ways selected from the following group: stamping, printing, painting, spraying, vaporizing.

9. Implant according to one of claims 1 to 8, **characterized in that** the central marking and/or at least one marking line is feelable.

10. Implant according to one of claims 1 to 9, **characterized in that** at least one marking line (3, 4, 5) has at least one of the features selected from the following group: continuous, interrupted, width in the range from 0.3 mm to 8.0 mm.

11. Implant according to one of claims 1 to 10, **characterized in that** the central marking (6) has one of the shapes selected from the following group: circle, rhombus, polygon, direction indicating shape.

12. Implant according to one of claims 1 to 11, **characterized in that** the central marking (6) and/or at least one marking line (3, 4, 5) contains a dye such that the central marking (6) and/or the at least one marking line (3, 4, 5) differs in colour from the basic structure (1, 2).

13. Implant according to claim 12, **characterized in that** the dye has at least one of the components selected from the following group: organic dyes, fluorescent dyes, inorganic dyestuff pigments, titanium dioxide, zirconium dioxide, iron pigments, magnetite.

14. Implant according to one of claims 1 to 13, **characterized in that** the basic structure (1, 2) is warp-knitted.

15. Implant according to one of claims 1 to 14, **characterized in that** the basic structure (1, 2) contains a non-resorbable or a slowly resorbable polymer, the basic structure preferably containing at least one polymer selected from the following group: polyacrylates, polymethacrylates, polyacrylamides, polyethylenes, polypropylenes, polyvinyl acetates, polyethylene-co-vinyl acetates, polyureas, polyesters, polyether esters, polyamides, polyimides, polyamino acids, pseudopolyamino acids, terephthalic acid-containing polyesters, partly fluorinated polyalkenes, perfluorinated polyalkenes, polyperfluoroethene, polyvinylidene fluoride, polycarbonates, polyarylether ketones, mixtures of the aforementioned substances, copolymers of the aforementioned substances.

16. Implant according to one of claims 1 to 15, **characterized in that** the basic structure (1, 2) contains a resorbable polymer, the basic structure preferably containing at least one polymer selected from the following group: polyhydroxy acids, polylactide, polyglycolide, polyhydroxy butyric acids, polydioxanones, polyhydroxy valeric acids, polyorthoesters, polyphosphazenes, poly-ε-caprolactones, polyphosphates, polyphosphonates, polyurethanes, polycyanoacrylates, mixtures of the aforementioned substances, copolymers of the aforementioned substances.

17. Implant according to one of claims 1 to 16, **characterized in that** the central marking and/or at least one marking line comprises a non-resorbable or a slowly resorbable polymer, the central marking and/or the at least one marking line preferably comprising at least one polymer selected from the following group: polyacrylates, polymethacrylates, polyacrylamides, polyethylenes, polypropylenes, polyvinyl acetates, polyethylene-co-vinyl acetates, polyureas; polyesters, polyether esters, polyamides, polyimides, polyamino acids, pseudopolyamino acids, terephthalic acid-containing polyesters, partly fluorinated polyalkenes, perfluorinated polyalkenes, polyperfluoroethene, polyvinylidene fluoride, polycarbonates, polyarylether ketones, mixtures of the aforementioned substances, copolymers of the aforementioned substances.

18. Implant according to one of claims 1 to 17, **characterized in that** the central marking (6) and/or at least one marking line (3, 4, 5) comprises a resorbable polymer, the central marking (6) and/or the at least one marking line (3, 4, 5) preferably containing at least one polymer selected from the following group: polyhydroxy acids, polylactide, polyglycolide, polyhydroxy butyric acids, polydioxanones, polyhydroxy valeric acids, polyorthoesters, polyphosphazenes, poly-ε-caprolactones, polyphosphates, polyphosphonates, polyurethanes, polycyanoacrylates, mixtures of the aforementioned substances, copolymers of the aforementioned substances.

19. Implant according to one of claims 1 to 18, **characterized in that** the back face of the implant has at least one marking.

20. Implant according to claim 19, **characterized in that** the at least one marking of the back face comprises at least one marking line.

21. Implant according to claim 19 or 20, **characterized in that** the pattern of markings on the back face is different from the pattern of markings defined in one of claims 1 to 18.

## Patentansprüche

1. Flächiges Implantat mit einer netzartigen Grundstruktur (1, 2) und einer Markierung (6) im Zentralbereich, wobei durch diese Zentralmarkierung (6) eine Markierungslinie (3) verläuft, wobei die Markierungslinie (3) gerade ist, **dadurch gekennzeichnet, dass** sich die Markierungslinie (3) bis zu den Rändern des Implantats erstreckt.

2. Implantat nach Anspruch 1, **gekennzeichnet durch** weitere Markierungslinien (4, 5).

3. Implantat nach Anspruch 2, **dadurch gekennzeichnet, dass** auf jeder Seite der durch die Zentralmarkierung (6) verlaufenden Markierungslinie (3) eine weitere Markierungslinie (4, 5) vorgesehen ist, die vorzugsweise parallel zu der durch die Zentralmarkierung (6) verlaufenden Markierungslinie (3) verläuft.

4. Implantat nach Anspruch 2 oder 3, **dadurch gekennzeichnet, dass** die durch die Zentralmarkierung verlaufende Markierungslinie eine größere Breite hat als die weiteren Markierungslinien.

5. Implantat nach einem der Ansprüche 1 bis 4, **dadurch gekennzeichnet, dass** die Zentralmarkierung (6) und/oder mindestens eine Markierungslinie (3, 4, 5) faden- oder garnartiges Material aufweist, das mit der Grundstruktur (1, 2) vorzugsweise auf eine der aus der folgenden Gruppe ausgewählten Arten verbunden ist: Wirken, Stricken, Sticken, Aufnähen, Knüpfen.

6. Implantat nach einem der Ansprüche 1 bis 5, **dadurch gekennzeichnet, dass** die Zentralmarkierung und/oder mindestens eine Markierungslinie flächiges Material aufweist, das mit der Grundstruktur vorzugsweise auf eine der aus der folgenden Gruppe ausgewählten Arten verbunden ist: Kleben, Aufschweißen, Nähen.

7. Implantat nach einem der Ansprüche 1 bis 6, **dadurch gekennzeichnet, dass** die Zentralmarkierung und/oder mindestens eine Markierungslinie mindestens einen Formkörper aufweist, vorzugsweise als Perle oder Röhrchen.

8. Implantat nach einem der Ansprüche 1 bis 7, **dadurch gekennzeichnet, dass** die Zentralmarkierung und/oder mindestens eine Markierungslinie ein Farbmittel aufweist, das vorzugsweise einen Farbstoff und ein Bindemittel enthält und das vorzugsweise auf eine der aus der folgenden Gruppe ausgewählten Arten aufgebracht ist: Stempeln, Drucken, Malen, Sprühen, Aufdampfen.

9. Implantat nach einem der Ansprüche 1 bis 8, **dadurch gekennzeichnet, dass** die Zentralmarkierung und/oder mindestens eine Markierungslinie tastbar ist.

10. Implantat nach einem der Ansprüche 1 bis 9, **dadurch gekennzeichnet, dass** mindestens eine Markierungslinie (3, 4, 5) mindestens eines der aus der folgenden Gruppe ausgewählten Merkmale hat: durchgehend, unterbrochen, Breite im Bereich von 0,3 mm bis 8,0 mm.

11. Implantat nach einem der Ansprüche 1 bis 10, **dadurch gekennzeichnet, dass** die Zentralmarkierung (6) eine der aus der folgenden Gruppe ausgewählten Formen hat: Kreis, Raute, Vieleck, richtungsangebende Form.

12. Implantat nach einem der Ansprüche 1 bis 11, **dadurch gekennzeichnet, dass** die Zentralmarkierung (6) und/oder mindestens eine Markierungslinie (3, 4, 5) einen Farbstoff aufweist, so dass sich die Zentralmarkierung (6) und/oder die mindestens eine Markierungslinie (3, 4, 5) farblich von der Grundstruktur (1, 2) unterscheidet.

13. Implantat nach Anspruch 12, **dadurch gekennzeichnet, dass** der Farbstoff mindestens eine der aus der folgenden Gruppe ausgewählten Komponenten hat: organische Farbstoffe, fluoreszierende Farbstoffe, anorganische Farbpigmente, Titandioxid, Zirkondioxid, Eisenpigmente, Magnetit.

14. Implantat nach einem der Ansprüche 1 bis 13, **dadurch gekennzeichnet, dass** die Grundstruktur (1, 2) gewirkt ist.

15. Implantat nach einem der Ansprüche 1 bis 14, **dadurch gekennzeichnet, dass** die Grundstruktur (1, 2) ein nicht resorbierbares oder ein langsam resorbierbares Polymer aufweist, wobei die Grundstruktur vorzugsweise mindestens ein aus der folgenden Gruppe ausgewähltes Polymer aufweist: Polyacrylate, Polymethacrylate, Polyacrylamide, Polyethylene, Polypropylene, Polyvinylacetate, Polyethylen-co-vinylacetate, Polyharnstoffe, Polyester, Polyetherester, Polyamide, Polyimide, Polyaminosäuren, Pseudopolyaminosäuren, Terephthalsäure enthaltende Polyester, teilfluorierte Polyalkene, perfluorierte Polyalkene, Polyperfluorethen, Polyvinylidenfluorid, Polycarbornate, Polyaryletherketone, Mischungen der vorgenannten Substanzen, Copolymere der vorgenannten Substanzen.

16. Implantat nach einem der Ansprüche 1 bis 15, **dadurch gekennzeichnet, dass** die Grundstruktur (1, 2) ein resorbierbares Polymer aufweist, wobei die Grundstruktur vorzugsweise mindestens ein aus der folgenden Gruppe ausgewähltes Polymer aufweist: Polyhydroxysäuren, Polylactid, Polyglykolid, Polyhydroxybuttersäuren, Polydioxanone, Polyhydroxyvaleriansäuren, Polyorthoester, Polyphosphazene, Poly-ε-caprolactone, Polyphosphate, Polyphosphonate, Polyurethane, Polycyanacrylate, Mischungen der vorgenannten Substanzen, Copolymere der vorgenannten Substanzen.

17. Implantat nach einem der Ansprüche 1 bis 16, **dadurch gekennzeichnet, dass** die Zentralmarkierung und/oder mindestens eine Markierungslinie ein nicht resorbierbares oder ein langsam resorbierbares Polymer aufweist, wobei die Zentralmarkierung und/oder die mindestens eine Markierungslinie vorzugsweise mindestens ein aus der folgenden Gruppe ausgewähltes Polymer aufweist: Polyacrylate, Polymethacrylate, Polyacrylamide, Polyethylene, Polypropylene, Polyvinylacetate, Polyethylen-co-vinylacetate, Polyharnstoffe, Polyester, Polyetherester, Polyamide, Polyimide, Polyaminosäuren, Pseudopolyaminosäuren, Terephthalsäure enthaltende Polyester, teilfluorierte Polyalkene, perfluorierte Polyalkene, Polyperfluorethen, Polyvinylidenfluorid, Polycarbonate, Polyaryletherketone, Mischungen der vorgenannten Substanzen, Copolymere der vorgenannten Substanzen.

18. Implantat nach einem der Ansprüche 1 bis 17, **dadurch gekennzeichnet, dass** die Zentralmarkierung (6) und/oder mindestens eine Markierungslinie (3, 4, 5) ein resorbierbares Polymer aufweist, wobei die Zentralmarkierung (6) und/oder die mindestens eine Markierungslinie (3, 4, 5) vorzugsweise mindestens ein aus der folgenden Gruppe ausgewähltes Polymer aufweist: Polyhydroxysäuren, Polylactid, Polyglykolid, Polyhydroxybuttersäuren, Polydioxanone, Polyhydroxyvaleriansäuren, Polyorthoester, Polyphosphazene, Poly-ε-caprolactone, Polyphosphate, Polyphosphonate, Polyurethane, Polycyanacrylate, Mischungen der vorgenannten Substanzen, Copolymere der vorgenannten Substanzen.

19. Implantat nach einem der Ansprüche 1 bis 18, **dadurch gekennzeichnet, dass** die Rückseite des Implantats mindestens eine Markierung aufweist.

20. Implantat nach Anspruch 19, **dadurch gekennzeichnet, dass** die mindestens eine Markierung der Rückseite mindestens eine Markierungslinie aufweist.

21. Implantat nach Anspruch 19 oder 20, **dadurch gekennzeichnet, dass** das Muster der Markierungen auf der Rückseite sich von dem Muster der in einem der Ansprüche 1 bis 18 definierten Markierungen unterscheidet.

## Revendications

1. Implant aréolaire ayant une structure de base de type maille (1, 2) et un marquage (6) dans sa zone centrale, une ligne de marquage (3) traversant ce marquage central (6), dans lequel la ligne de marquage (3) est droite, **caractérisé en ce que** la ligne de marquage (3) s'étend en montant jusqu'aux bords de l'implant.

2. Implant selon la revendication 1, **caractérisé par** d'autres lignes de marquage (4, 5).

3. Implant selon la revendication 2, **caractérisé en ce que**, sur chaque côté de la ligne de marquage (3) traversant le marquage central (6), est prévue une autre ligne de marquage (4, 5) qui passe de préférence parallèlement à la ligne de marquage (3) traversant le marquage central (6).

4. Implant selon la revendication 2 ou 3, **caractérisé en ce que** la ligne de marquage traversant le marquage central a une largeur supérieure aux autres lignes de marquage.

5. Implant selon l'une des revendications 1 à 4, **caractérisé en ce que** le marquage central (6) et/ou au moins une ligne de marquage (3, 4, 5) comprennent un matériau de type fil qui est relié à la structure de base (1, 2) de préférence d'une des manières choisies dans le groupe suivant : tricotage chaîne, tricotage trame, broderie, couture, nouage.

6. Implant selon l'une des revendications 1 à 5, **caractérisé en ce que** le marquage central et/ou au moins une ligne de marquage contient le matériau aréolaire qui est relié à la structure de base de préférence d'une des manières choisies dans le groupe suivant : collage, soudage, couture.

7. Implant selon l'une des revendications 1 à 6, **caractérisé en ce que** le marquage central et/ou au moins une ligne de marquage comprend au moins un corps façonné, de préférence sous la forme d'une perle ou d'un petit tube.

8. Implant selon l'une quelconque des revendications 1 à 7, **caractérisé en ce que** le marquage central et/ou au moins une ligne de marquage contient(contiennent) un agent colorant, qui comprend de préférence un colorant ou un liant et qui est de préférence appliqué de l'une des manières choisies dans le groupe suivant : estampage, impression, peinture, pulvérisation, vaporisation.

9. Implant selon l'une des revendications 1 à 8, **caractérisé en ce que** le marquage central et/ou au moins une ligne de marquage est palpable.

10. Implant selon l'une des revendications 1 à 9, **caractérisé en ce qu'**au moins une ligne de marquage (3, 4, 5) possède au moins une des caractéristiques choisies dans le groupe suivant : continue, interrompue, largeur dans la plage de 0,3 mm à 8,0 mm.

11. Implant selon l'une des revendications 1 à 10, **caractérisé en ce que** le marquage central (6) a l'une des formes choisies dans le groupe suivant : cercle, losange, polygone, forme indiquant la direction.

12. Implant selon l'une des revendications 1 à 11, **caractérisé en ce que** le marquage central (6) et/ou au moins une ligne de marquage (3, 4, 5) contient(contiennent) un colorant de telle sorte que le marquage central (6) et/ou l'au moins une ligne de marquage (3, 4, 5) diffère(nt) par la couleur de la structure de base (1, 2).

13. Implant selon la revendication 12, **caractérisé en ce que** le colorant possède au moins un des composants choisis dans le groupe suivant : colorants organiques, colorants fluorescents, pigments de colorants inorganiques, dioxyde de titane, dioxyde de zirconium, pigments de fer, magnétite.

14. Implant selon l'une des revendications 1 à 13, **caractérisé en ce que** la structure de base (1, 2) est tricotée en chaîne.

15. Implant selon l'une des revendications 1 à 14, **caractérisé en ce que** la structure de base (1, 2) contient un polymère non résorbable ou à résorption lente, la structure de base contenant de préférence au moins un polymère choisi dans le groupe suivant : polyacrylates, polyméthacrylates, polyacrylamides, polyéthylènes, polypropylènes, acétates de polyvinyle, acétates de polyéthylène-co-vinyle, polyrésines, polyesters, esters de polyéther, polyamides, polyimides, acides polyaminés, acides pseudopolyaminés, polyesters contenant de l'acide térephtalique, polyalcènes partiellement fluorés, polyalcènes perfluorés, polyperfluoréthène, polyfluorure de vinylidène, polycarbonates, polyaryléther cétones, mélanges des substances susmentionnées, copolymères des substances susmentionnées.

16. Implant selon l'une des revendications 1 à 15, **caractérisé en ce que** la structure de base (1, 2) contient un polymère résorbable, la structure de base contenant de préférence au moins un polymère choisi dans le groupe suivant : acides polyhydroxy, polylactide, polyglycolide, acides polyhydroxy butyriques, polydioxanones, acides polyhydroxy valériques, polyorthoesters, polyphosphazènes, poly-ε-caprolactones, polyphosphates, polyphosphonates, polyuréthanes, polycyanoacrylates, mélanges des substances susmentionnées, copolymères des substances susmentionnées.

17. Implant selon l'une des revendications 1 à 16, **caractérisé en ce que** le marquage central et/ou au moins une ligne de marquage comprend un polymère non résorbable ou à résorption lente, le marquage central et/ou l'au moins une ligne de marquage comprenant de préférence au moins un polymère choisi dans le groupe suivant : polyacrylates, polyméthacrylates, polyacrylamides, polyéthylènes, polypropylènes, acétates de polyvinyle, acétates de polyéthylène-co-vinyle, polyrésines, polyesters, esters de polyéther, polyamides, polyimides, acides polyaminés, acides pseudopolyaminés, polyesters contenant de l'acide térephtalique, polyalcènes partiellement fluorés, polyalcènes perfluorés, polyperfluoréthène, polyfluorure de vinylidène, polycarbonates, polyaryléther cétones, mélanges des substances susmentionnées, copolymères des substances susmentionnées.

18. Implant selon l'une des revendications 1 à 17, **caractérisé en ce que** le marquage central (6) et/ou au moins une ligne de marquage (3, 4, 5) comprend(comprennent) un polymère résorbable, le marquage central (6) et/ou l'au moins une ligne de marquage (3, 4, 5) contenant de préférence au moins un polymère choisi dans le groupe suivant : acides polyhydroxy, polylactide, polyglycolide, acides polyhydroxy butyriques, polydioxanones, acides polyhydroxy valériques, polyorthoesters, polyphosphazènes, poly-ε-caprolactones, polyphosphates, polyphosphonates, polyuréthanes, polycyanoacrylates, mélanges des substances susmentionnées, copolymères des substances susmentionnées.

19. Implant selon l'une des revendications 1 à 18, **caractérisé en ce que** la face arrière de l'implant possède au moins un marquage.

20. Implant selon la revendication 19, **caractérisé en ce que** l'au moins un marquage de la face arrière comprend au moins une ligne de marquage.

21. Implant selon la revendication 19 ou 20, **caractérisé en ce que** le motif des marquages sur la face arrière est différent du motif de marquages défini dans l'une des revendications 1 à 18.
